# EUROPEAN PATENT SPECIFICATION

(11) **EP 3 756 709 B1**
(45) Date of publication and mention of the grant of the patent: **21.02.2024**
(21) Application number: 18918743.8
(22) Date of filing: 26.06.2018
(51) Int. Cl.: B05B 17/00, A61M 11/00, A61M 15/00

(54) **INTEGRATED SEALED MICRO-MESH NEBULIZING MODULE**
VERNEBLUNGSMODUL MIT INTEGRIERTEM VERSIEGELTEM FEINMASCHIGEM GITTER
MODULE DE NÉBULISATION À MICRO-MAILLES SCELLÉ DE MANIÈRE ÉTANCHE, INTÉGRÉ

(30) Priority: 18.05.2018 CN 201820743644 U
(43) Date of publication of application: 30.12.2020
(73) Proprietor: Feellife Health INC., Shenzhen (CN)
(72) Inventor: SONG, Xuefeng, Shenzhen, Guangdong 518000 (CN); HUA, Jian, Shenzhen, Guangdong 518000 (CN)
(74) Representative: Arnold & Siedsma
(86) International application number: PCT/CN2018/092923
(87) International publication number: WO 2019/218428

(56) References cited:
- CN-A- 105 311 718
- CN-A- 106 178 202
- CN-U- 205 181 935
- CN-U- 205 198 623
- CN-U- 206 934 411
- US-A1- 2005 081 844
- US-A1- 2006 207 591
- US-A1- 2007 267 010
- US-A1- 2015 102 124
- US-A1- 2015 238 993
- US-A1- 2015 306 334

## Description

### TECHNICAL FIELD

The present invention belongs to the field of electronic nebulizers, and more particularly, relates to an integrated sealed micro-mesh nebulization module.

### BACKGROUND

Nebulizer treatment has a principle of nebulizing a medicine into micro droplets, and the atomized liquid is inhaled into a human body by respiration to achieve a therapeutic effect. In this way, not only pain of taking the medicine is alleviated, but also waste of the medicine caused by a process of taking the medicine is avoided, and the therapeutic effect of the medicine is also able to be fully exerted. A nebulizer has many functions in medical treatment, comprising anti-inflammation, cough relief, phlegm elimination, bronchospasm relief, smoothing of airway and improvement of ventilation, so that a patient may not feel dyspnea during treatment. The nebulizer may also be used before and after chest surgery to prevent respiratory tract infection, so that the surgery may have good a result, and the nebulizer may be used in treatment of lung cancer with an anticancer medicine. A micro-mesh nebulization sheet is an important part of a micro-mesh nebulizer, which is mainly made of a metal mesh and a ceramic material. The micro-mesh nebulization sheet oscillates at a certain frequency under driving of a circuit, so that a ceramic vibrating reed oscillates resonantly, thus driving the metal mesh to oscillate at a high speed. Therefore, a liquid medicine passes through numerous micro-mesh openings in the metal mesh and is quickly ejected to form countless atomized micro particles. However, the nebulization sheet in the market now usually needs to be purchased separately, and a series of problems such as clamping and fixing, sealing and waterproofing, circuit welding and so on may be encountered during assembly, thus greatly affecting a universality of the nebulization sheet. If the nebulization sheet needs to be disassembled, such as cleaning and sterilization, a nozzle and other connecting parts must be disassembled before performing routine maintenance on the nebulizer.

CN205181935U discloses a medical atomizer, including putting coyote hole, atomizer and handheld seat, put the coyote hole setting at a handheld seat top, put coyote hole one side and be equipped with atomizing mouthful and be connected with the atomizer, the bottom surface of putting the coyote hole is the V-arrangement face, and V-arrangement shape of face one -tenth water conservancy diversion structure, and the curb of V-arrangement face is facing to the atomizing mouth.

US20070267010A1 discloses a method of treating a patient with a pulmonary disease, where the method includes delivering a dose of aerosolized medicament intermittently to a ventilator circuit coupled to the respiratory system of the patient.

US20150102124A1 discloses a nozzle replaceable atomizer with automatic abnormality detecting function is provided. In the atomizer, when a detachable spray head is assembled to a main machine, a microcomputer serves to automatically set a piezoelectric element at an optimal operating frequency.

### SUMMARY

In view of the above problem, the present invention is intended to provide an integrated sealed micro-mesh nebulization module, which is universal and convenient to mount, and facilitates sterilization and cleaning.

In order to achieve the above objective, the present invention provides an integrated sealed micro-mesh nebulization module, which comprises a nebulization device and a nozzle mechanism disposed on the nebulization device, wherein the nebulization device is detachably connected with the nozzle mechanism disposed on the nebulization device. The nebulization module comprises an annular base, a lower shell disposed in the annular base, an upper shell disposed on the lower shell, a micro-mesh nebulization sheet disposed on the lower shell, a first sealing ring and a second sealing ring respectively disposed on both sides of the micro-mesh nebulization sheet, and a metal contact disposed between the lower shell and the upper shell, with one end connected with the micro-mesh nebulization sheet through a wire, and the other end extending to an exterior of the lower shell and connected with an external driving circuit. A first through hole is disposed in a center of the upper shell, and a second through hole corresponding to the first through hole is disposed in a center of the lower shell. The nozzle mechanism comprises a bottom cover disposed on one side of the upper shell in the annular base, a tubular spout disposed on the bottom cover, and gas outlets for discharging excess atomized gas respectively disposed on the bottom covers at upper and lower sides of the spout. The bottom cover is provided with a central hole corresponding to the first through hole. Therefore, the upper shell, the first sealing ring, the micro-mesh nebulization sheet, the second sealing ring and the lower shell are connected in sequence to form a sealed and closed state, so that gas generated by liquid flowing through the second through hole on the micro-mesh nebulization sheet may be sprayed out from the spout through the first through hole and the central hole in sequence, and excess gas is discharged from the gas outlet. In addition, an I-shaped sealing ring is used as the second sealing ring.

In some embodiments, an edge of the upper shell is provided with an annular bulge serving as an ultrasonic line.

In some embodiments, the lower shell is provided with a first threaded hole and a second threaded hole for connecting the bottom cover.

In some embodiments, the gas outlet is an arc-shaped port, and a shielding portion is disposed at an edge of the port for discharging gas inside the spout and preventing an external foreign matter from entering the spout. Therefore, not only the atomized gas generated inside the spout may be discharged to the outside from the gas outlet, but also the external foreign matter may be prevented from entering the spout to cause pollution through this hidden-type design.

According to the invention, a first gas channel is further provided at an inner wall of the spout, the nebulization device is also provided with a second gas channel corresponding to the first gas channel, and the first gas channel is communicated with the second gas channel.

In some embodiments, a limiting groove and a limiting bulge are respectively provided at a contact surface between the upper shell and the lower shell.

The present invention has the beneficial effects of being universal and convenient to mount, and facilitating sterilization and cleaning. Since the upper shell, the first sealing ring, the micro-mesh nebulization sheet, the second sealing ring and the lower shell are connected, extruded and fixed together in sequence by ultrasonic welding, the micro-mesh nebulization sheet is electrically connected with the metal contact, and the metal contact is externally connected with the driving circuit, so that the micro-mesh nebulization sheet and the components thereof are modularized to form an nebulizing unit, thus improving a universality of the nebulization sheet. Moreover, an extension portion of the lower shell is provided with the first threaded hole for connecting the nebulizing unit with the nozzle; the bottom cover of the nozzle is provided with the second threaded hole for connecting the nozzle with an nebulizer body; and the gas outlet is disposed at an adjacent position between the spout and the bottom cover for discharging the excess atomized gas. In this way, the nebulizing unit is mounted on the nozzle to form the integrated nebulization module, and then the nebulization module is mounted on the nebulizer body, so that the nebulizing unit can be disassembled by disassembling the nozzle, thus facilitating cleaning and sterilization of the nebulization sheet. In addition, the I-shaped sealing ring is used as the second sealing ring, so that the corresponding sealing ring can be omitted at a contact portion between the lower shell of the nebulizing unit and the nebulizer body, and a structure of a joint among the components is simpler while being waterproof, so as to prevent misplacement, dropping, loss and so on of the parts. Therefore, the present invention not only improves the universality of the nebulization sheet, but also can disassemble the nebulizing unit by disassembling the nozzle, thus further facilitating cleaning and sterilization of the nebulization sheet.

### BRIEF DESCRIPTION OF THE DRAWINGS

FIG. 1 is a structure diagram of the embodiment 1, not encompassed by the wording of the claims;
FIG. 2 is a structure diagram of a front view of the embodiment 1, not encompassed by the wording of the claims;
FIG. 3 is a structure diagram of a rear view of the embodiment 1, not encompassed by the wording of the claims;
FIG. 4 is a structure diagram of an A-A cross-section in FIG. 2;
FIG. 5 is a structure diagram of a B-B cross-section in FIG. 2;
FIG. 6 is a structure diagram of the embodiment 2;
FIG. 7 is a structure diagram of a front view of the embodiment 2;
FIG. 8 is a structure diagram of a rear view of the embodiment 2; and
FIG. 9 is a structure diagram of a C-C cross-section in FIG. 8.

### DETAILED DESCRIPTION

The present invention is further described in detail hereinafter with reference to the accompanying drawings.

### Embodiment 1 not encompassed by the wording of the claims

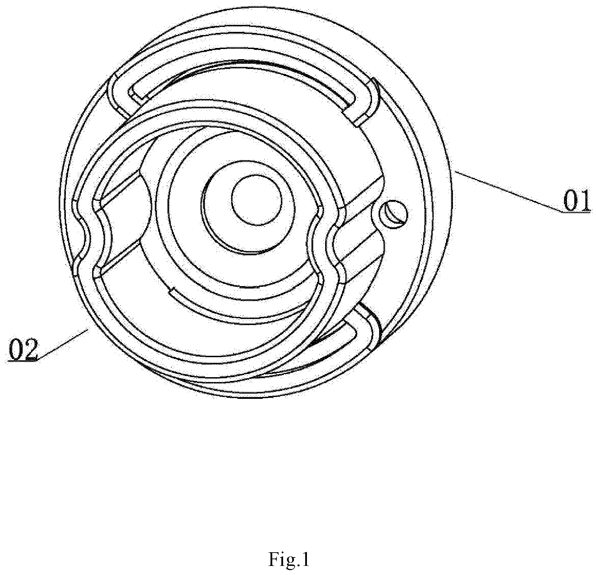

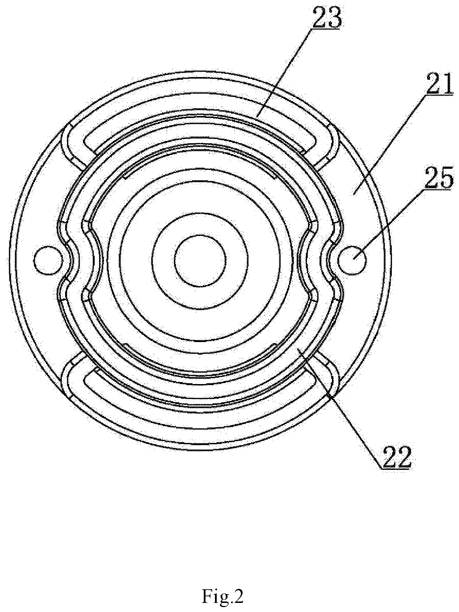

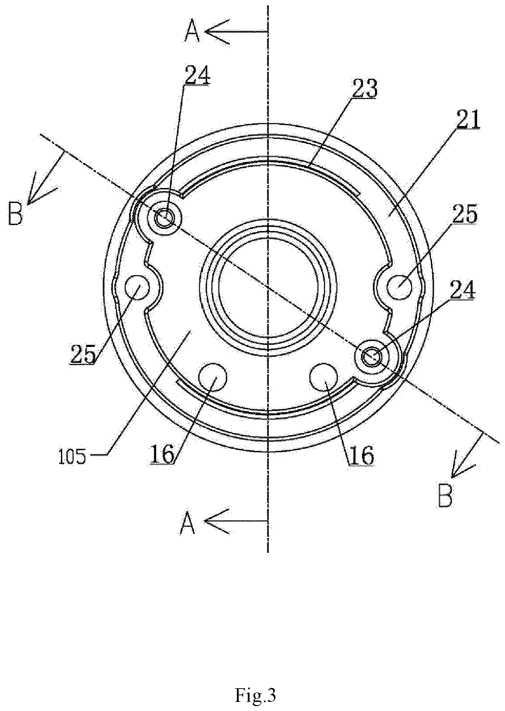

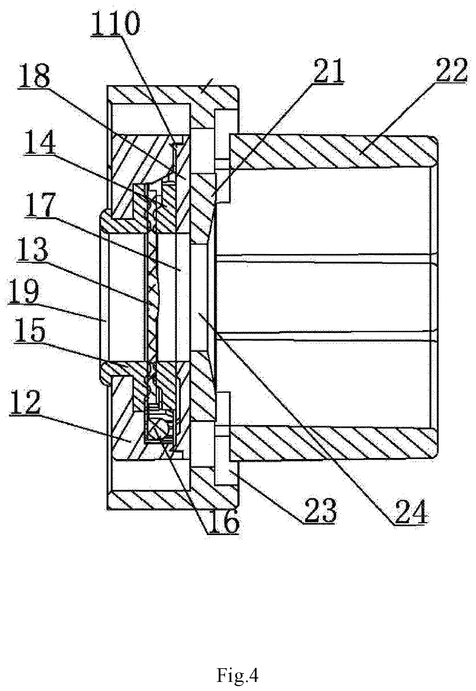

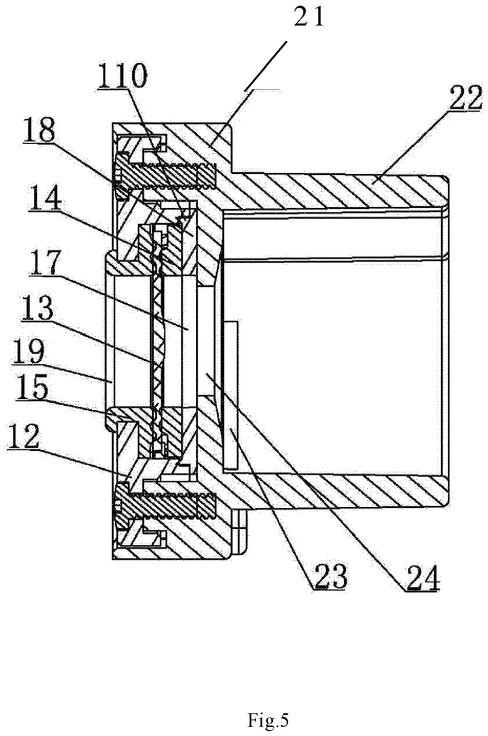

As shown in FIG. 1 to FIG. 5, an integrated sealed micro-mesh nebulization module comprises a nebulization device 01 and a nozzle mechanism 02 disposed on the nebulization device 01. The nebulization device 01 is detachably connected with the nozzle mechanism 02 disposed on the nebulization device 01. The nebulization device 01 comprises an annular base 11, a lower shell 12 disposed in the annular base 11, an upper shell 18 disposed on the lower shell 12, a micro-mesh nebulization sheet 13 disposed on the lower shell 12, a first sealing ring 14 and a second sealing ring 15 respectively disposed on both sides of the micro-mesh nebulization sheet 13, and a metal contact 16 disposed between the lower shell 12 and the upper shell 18, with one end connected with the micro-mesh nebulization sheet 13 through a wire, and the other end extending to an exterior of the lower shell 12 and connected with an external driving circuit. A first through hole 17 is disposed in a center of the upper shell 18, and a second through hole 19 corresponding to the first through hole 17 is disposed in a center of the lower shell 12. The nozzle mechanism 02 comprises a bottom cover 21 disposed on one side of the upper shell 18 in the annular base 11, a tubular spout 22 disposed on the bottom cover 21, and gas outlets 23 for discharging excess atomized gas respectively disposed on the bottom covers 21 at upper and lower sides of the spout 22. The bottom cover 21 is provided with a central hole 24 corresponding to the first through hole 17. The upper shell 18, the first sealing ring 14, the micro-mesh nebulization sheet 13, the second sealing ring 15 and the lower shell 12 are connected in sequence to form a sealed and closed state, so that gas generated by liquid flowing through the second through hole 19 on the micro-mesh nebulization sheet 13 may be sprayed out from the spout 22 through the first through hole 17 and the central hole 24 in sequence, and excess gas is discharged from the gas outlet 23. In addition, an I-shaped sealing ring is used as the second sealing ring 15. The above spout 22 may have various shapes with a hollow interior. On the basis of making the spout 22 into a cylinder, an inwardly concave arc is designed at a corresponding position. The gas outlet may also be disposed at any position outside a wall of the spout 22 or at any adjacent position of the spout 22 and the bottom cover 21, and may have various shapes. An edge of the upper shell 18 is provided with an annular bulge 110 serving as an ultrasonic line 110. The lower shell 12 is provided with a first threaded hole 24 and a second threaded hole 25 for connecting the bottom cover 21. The gas outlet 23 is an arc-shaped opening, and a convex shielding portion is disposed at the edge of the gas outlet 23 to shield the gas outlet 23. Not only the atomized gas generated inside the spout may be discharged to the outside from the gas outlet 23, but also the external foreign matter may be prevented from entering the spout to cause pollution through this hidden design. A limiting groove and a limiting bulge are respectively provided at a contact surface between the upper shell 18 and the lower shell 12, thus being closed more tightly.

During application, the upper shell 18, the first sealing ring 14, the micro-mesh nebulization sheet 13, the second sealing ring 15 and the lower shell 12 are connected, extruded and fixed together in sequence by ultrasonic welding, the micro-mesh nebulization sheet 13 is electrically connected with the metal contact 16, and the metal contact 16 is externally connected with the driving circuit, so that the micro-mesh nebulization sheet 13 and the components thereof are modularized to form an nebulizing unit, thus improving a use universality of the nebulization sheet. Moreover, an extension portion of the lower shell 12 is provided with the first threaded hole 24 for connecting the nebulizing unit with the nozzle. The bottom cover 21 of the nozzle is provided with the second threaded hole 25 for connecting the nozzle with an nebulizer body. The gas outlet 23 is disposed at an adjacent position between the spout 22 and the bottom cover 21 for discharging the excess atomized gas. In this way, the nebulizing unit is mounted on the nozzle to form the integrated nebulization module, and then the nebulization module is mounted on the nebulizer body, so that the nebulizing unit may be disassembled by disassembling the nozzle, thus facilitating cleaning and sterilization of the nebulization sheet. In addition, the I-shaped sealing ring is used as the second sealing ring 15, so that the corresponding sealing ring may be omitted at a contact portion between the lower shell 12 of the nebulizing unit and the nebulizer body, and a structure of a joint among the components is simpler while being waterproof, so as to prevent misplacement, dropping, loss and so on of the parts.

### Embodiment 2

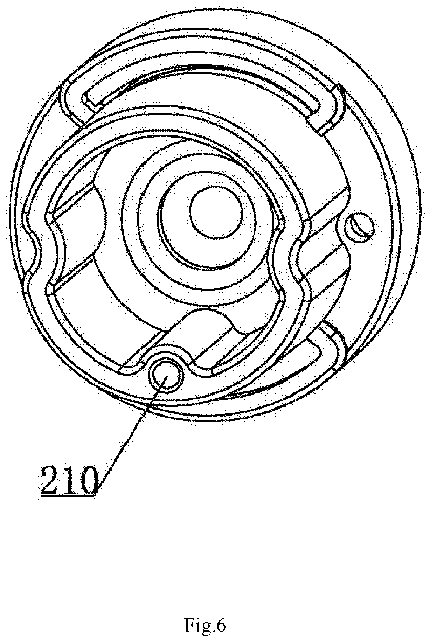

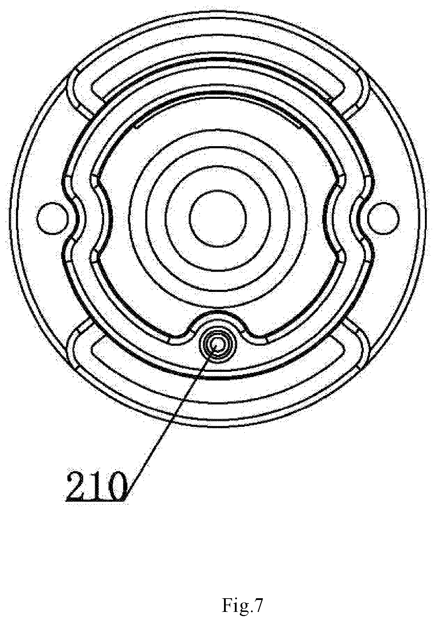

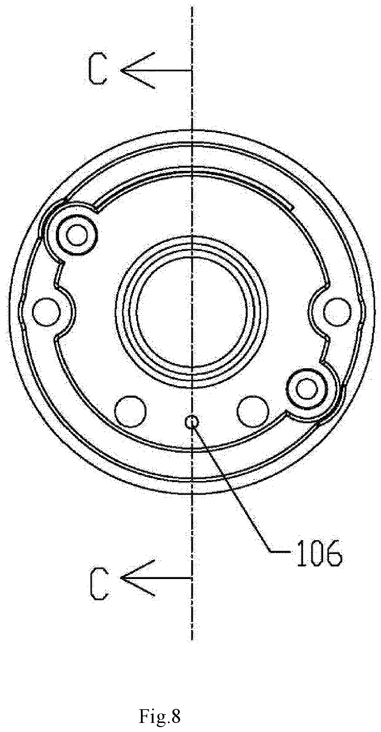

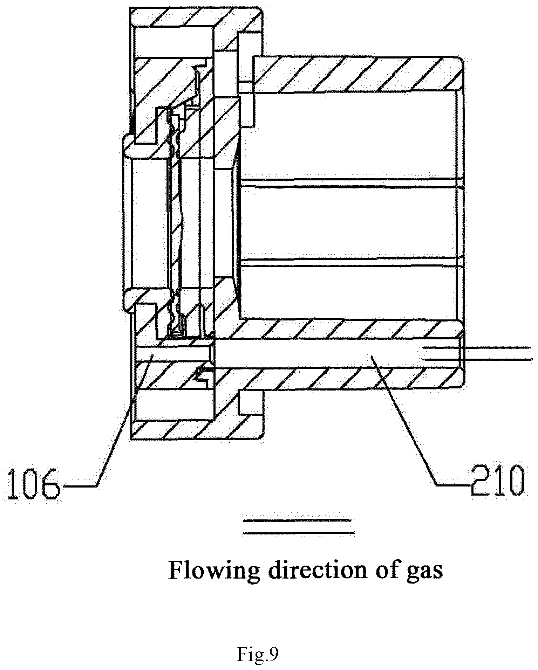

As shown in FIG. 6 to FIG. 9, an integrated sealed micro-mesh nebulization module is added with a first gas channel 210 on an inner wall of a spout 22 on the basis of a structure of the embodiment 1. The first gas channel 210 may be disposed at any position on the inner wall of the spout 22. In the embodiment 2, the first gas channel 210 is disposed at a central line of a bottom portion of the inner wall. Correspondingly, the nebulization device 01 is provided with a second gas channel 106 corresponding to the first gas channel 210, and the second gas channel 106 is formed by perforated portions of moulds of an upper shell 18 and a lower shell 12. The second gas channel 106 is communicated with the first gas channel 210, and provides an independent gas channel for the nebulization module, so that an air flow generated by expiration and inspiration of a user is not interfered by nebulization of an nebulizer, thus being convenient for detecting a pressure and/or a flow rate of the air flow respired by the user. In the embodiment, since a nozzle mechanism 02 is added with the first gas channel 210, a gas outlet 23 below the spout 22 can be omitted. Since the nebulization device 01 is provided with the second gas channel 106, sizes of components such as a micro-mesh nebulization sheet and a sealing ring are adjusted.

The above embodiments are only some embodiments of the present invention. Those of ordinary skills in the art may further make several modifications and improvements without departing from the scope of protection of the present invention. In particular, the position of the gas channel may be designed to other places of the shell. These modifications and improvements all fall within the scope of protection of the present invention as defined by the appended claims.

## Claims

1. An integrated sealed micro-mesh nebulization module, comprising:
a nebulization device (01) and a nozzle mechanism (02) disposed on the nebulization device (01), wherein the nozzle mechanism (02) disposed on the nebulization device is detachably connected with the nebulization device (01);
wherein the nebulization device (01) comprises an annular base (11), a lower shell (12) disposed in the annular base (11), an upper shell (18) disposed on the lower shell (12), a micro-mesh nebulization sheet (13) disposed on the lower shell (12), a first sealing ring (14) and a second sealing ring (15) respectively disposed on both sides of the micro-mesh nebulization sheet (13), and a metal contact (16) disposed between the lower shell (12) and the upper shell (18), with one end connected with the micro-mesh nebulization sheet (13) through a wire, and the other end extending to an exterior of the lower shell (12) and connected with an external driving circuit;
wherein a first through hole (17) is disposed in a center of the upper shell (18), and a second through hole (19) corresponding to the first through hole (17) is disposed in a center of the lower shell (12);
wherein the nozzle mechanism (02) comprises a bottom cover (21) disposed on one side of the upper shell (18) in the annular base (11), a tubular spout (22) disposed on the bottom cover (21), and gas outlets (23) for discharging excess atomized gas respectively disposed on the bottom cover (21) at upper and lower sides of the spout; and wherein the bottom cover (21) is provided with a central hole (24) corresponding to the first through hole (17); and
**characterized in that** a first gas channel (210) is further provided at an inner wall of the spout, the nebulization device (01) is further provided with a second gas channel (106) corresponding to the first gas channel, and the first gas channel is communicated with the second gas channel.

2. The integrated sealed micro-mesh nebulization module of claim 1, wherein an edge of the upper shell (18) is provided with an annular bulge serving as an ultrasonic line.

3. The integrated sealed micro-mesh nebulization module of claim 2, wherein the lower shell (12) is provided with a first threaded hole and a second threaded hole for connecting the bottom cover.

4. The integrated sealed micro-mesh nebulization module of claim 1, 2 or 3, wherein the gas outlet (23) is an arc-shaped port, and a shielding portion is disposed at an edge of the port for discharging gas inside the spout and preventing an external foreign matter from entering the spout.

5. The integrated sealed micro-mesh nebulization module of claim 1, wherein a limiting groove and a limiting bulge are respectively provided at a contact surface between the upper shell (18) and the lower shell (12).

## Patentansprüche

1. Integriertes abgedichtetes Mikromaschenzerstäubungsmodul, das Folgendes umfasst:
eine Zerstäubungsvorrichtung (01) und einen Düsenmechanismus (02), der auf der Zerstäubungsvorrichtung (01) angeordnet ist, wobei der auf der Zerstäubungsvorrichtung angeordnete Düsenmechanismus (02) lösbar mit der Zerstäubungsvorrichtung (01) verbunden ist;
wobei die Zerstäubungsvorrichtung (01) eine ringförmige Basis (11), eine untere Hülse (12), die in der ringförmigen Basis (11) angeordnet ist, eine obere Hülse (18), die auf der unteren Hülse (12) angeordnet ist, eine Mikromaschenzerstäubungsfolie (13), die auf der unteren Hülse (12) angeordnet ist, einen ersten Dichtring (14) und einen zweiten Dichtring (15), die jeweils auf beiden Seiten der Mikromaschenzerstäubungsfolie (13) angeordnet sind, und einen Metallkontakt (16), der zwischen der unteren Hülse (12) und der oberen Hülse (18) angeordnet ist und dessen ein Ende über einen Draht mit der Mikromaschenzerstäubungsfolie (13) verbunden ist und dessen anderes Ende sich zu einer Außenseite der unteren Hülse (12) erstreckt und mit einer äußeren Ansteuerschaltung verbunden ist;
wobei ein erstes Durchgangsloch (17) in einer Mitte der oberen Hülse (18) angeordnet ist und ein zweites Durchgangsloch (19), das dem ersten Durchgangsloch (17) entspricht, in einer Mitte der unteren Hülse (12) angeordnet ist;
wobei der Düsenmechanismus (02) eine untere Abdeckung (21), die auf einer Seite der oberen Hülse (18) in der ringförmigen Basis (11) angeordnet ist, eine rohrförmige Auslassrinne (22) die auf der unteren Abdeckung (21) angeordnet ist, und Gasauslässe (23) zum Ausgeben von überschüssigem zerstäubtem Gas, die jeweils auf der unteren Abdeckung (21) auf einer oberen und einer unteren Seite der Auslassrinne angeordnet sind; und wobei die untere Abdeckung (21) mit einem mittiges Loch (24) versehen ist, das dem ersten Durchgangsloch (17) entspricht; und
**dadurch gekennzeichnet, dass** ferner an einer Innenwand der Auslassrinne ein erster Gaskanal (210) bereitgestellt ist, die Zerstäubungsvorrichtung (01) ferner mit einem zweiten Gaskanal (106) versehen ist, der dem ersten Gaskanal entspricht, und der erste Gaskanal mit dem zweiten Gaskanal kommuniziert.

2. Integriertes abgedichtetes Mikromaschenzerstäubungsmodul nach Anspruch 1, wobei eine Kante der oberen Hülse (18) mit einer ringförmigen Wölbung versehen ist, die als eine Ultraschalllinie dient.

3. Integriertes abgedichtetes Mikromaschenzerstäubungsmodul nach Anspruch 2, wobei die untere Hülse (12) zum Verbinden der unteren Abdeckung mit einem ersten Gewindeloch und einem zweiten Gewindeloch versehen ist.

4. Integriertes abgedichtetes Mikromaschenzerstäubungsmodul nach Anspruch 1, 2 oder 3, wobei der Gasauslass (23) ein bogenförmiger Port ist und zum Ausgeben von Gas in der Auslassrinne und um zu verhindern, dass äußere Fremdkörper in die Auslassrinne eintreten, an einer Kante des Ports ein Abschirmabschnitt vorgesehen ist.

5. Integriertes abgedichtetes Mikromaschenzerstäubungsmodul nach Anspruch 1, wobei an einer Kontaktfläche zwischen der oberen Hülse (18) und der unteren Hülse (12) eine Begrenzungsnut bzw. eine Begrenzungswölbung vorgesehen sind.

## Revendications

1. Module de nébulisation à micro-mailles intégré scellé de manière étanche comprenant :
un dispositif de nébulisation (01) et un mécanisme de buse (02) disposé sur le dispositif de nébulisation (01), dans lequel le mécanisme de buse (02) disposé sur le dispositif de nébulisation est raccordé, de manière détachable, avec le dispositif de nébulisation (01) ;
dans lequel le dispositif de nébulisation (01) comprend une base annulaire (11), une coque inférieure (12) disposée dans la base annulaire (11), une coque supérieure (18) disposée sur la coque inférieure (12), une feuille de nébulisation à micro-mailles (13) disposée sur la coque inférieure (12), une première bague d'étanchéité (14) et une seconde bague d'étanchéité (15) respectivement disposées des deux côtés de la feuille de nébulisation à micro-mailles (13), et un contact en métal (16) disposé entre la coque inférieure (12) et la coque supérieure (18), avec une extrémité raccordée avec la feuille de nébulisation à micro-mailles (13) par le biais d'un fil, et l'autre extrémité s'étendant vers un extérieur de la coque inférieure (12) et raccordée avec un circuit d'entraînement externe ;
dans lequel un premier trou débouchant (17) est disposé dans un centre de la coque supérieure (18), et un second trou débouchant (19) correspondant au premier trou débouchant (17) est disposé dans un centre de la coque inférieure (12) ;
dans lequel le mécanisme de buse (02) comprend un couvercle inférieur (21) disposé d'un côté de la coque supérieure (18) dans la base annulaire (11), un bec verseur tubulaire (22) disposé sur le couvercle inférieur (21), et des sorties de gaz (23) pour décharger le gaz atomisé en excès respectivement disposées sur le couvercle inférieur (21) au niveau des côtés supérieur et inférieur du bec verseur ; et dans lequel le couvercle inférieur (21) est prévu avec un trou central (24) correspondant au premier trou débouchant (17) ; et
**caractérisé en ce qu'**un premier canal de gaz (210) est en outre prévu au niveau d'une paroi interne du bec verseur, le dispositif de nébulisation (01) est en outre prévu avec un second canal de gaz (106) correspondant au premier canal de gaz, et le premier canal de gaz est en communication avec le second canal de gaz.

2. Module de nébulisation à micro-mailles intégré scellé de manière étanche selon la revendication 1, dans lequel un bord de la coque supérieure (18) est prévu avec un renflement annulaire servant de ligne ultrasonore.

3. Module de nébulisation à micro-mailles intégré scellé de manière étanche selon la revendication 2, dans lequel la coque inférieure (12) est prévue avec un premier trou fileté et un second trou fileté pour raccorder le couvercle inférieur.

4. Module de nébulisation à micro-mailles intégré scellé de manière étanche selon la revendication 1, 2 ou 3, dans lequel la sortie de gaz (23) est un orifice en forme d'arc, et une partie de protection est disposée au niveau d'un bord de l'orifice pour décharger le gaz à l'intérieur du bec verseur et empêcher un corps étranger externe d'entrer dans le bec verseur.

5. Module de nébulisation à micro-mailles intégré scellé de manière étanche selon la revendication 1, dans lequel une rainure de limitation et un renflement de limitation sont respectivement prévus au niveau d'une surface de contact entre la coque supérieure (18) et la coque inférieure (12).
